(19) Europäisches Patentamt
European Patent Office
Office européen des brevets

(11) EP 2 771 072 B1

(12) **EUROPEAN PATENT SPECIFICATION**

(45) Date of publication and mention of the grant of the patent:
**13.12.2017 Bulletin 2017/50**

(21) Application number: **12770470.8**

(22) Date of filing: **03.10.2012**

(51) Int Cl.:
***A61Q 17/04*** *(2006.01)* ***A61K 8/25*** *(2006.01)*
***A61K 8/37*** *(2006.01)* ***A61K 8/58*** *(2006.01)*
***A61K 8/89*** *(2006.01)* ***A61K 8/92*** *(2006.01)*

(86) International application number:
**PCT/EP2012/069535**

(87) International publication number:
**WO 2013/060559 (02.05.2013 Gazette 2013/18)**

(54) **AN AQUEOUS PHOTO-PROTECTIVE PERSONAL CARE COMPOSITION**

WÄSSRIGE KÖRPERPFLEGEZUSAMMENSETZUNGEN MIT LICHTSCHUTZ

COMPOSITION PHOTO-PROTECTRICE AQUEUSE DE SOINS PERSONNELS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2011 IN MM30412011**
**23.01.2012 EP 12152054**

(43) Date of publication of application:
**03.09.2014 Bulletin 2014/36**

(73) Proprietors:
- **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**
- **Unilever PLC**
**London, Greater London EC4Y 0DY (GB)**
Designated Contracting States:
**CY GB IE MT**

(72) Inventors:
- **VAIDYA, Ashish,Anant**
**Bangalore 560 066 (IN)**
- **GHOSH, Nilmoni**
**741 101 West Bengal (IN)**

(74) Representative: **Corsten, Michael Allan**
**Unilever N.V.**
**Unilever Patent Group**
**Olivier van Noortlaan 120**
**3133 AT Vlaardingen (NL)**

(56) References cited:
**EP-A1- 1 391 193**
**EP-A1- 1 535 938**
**EP-A1- 2 100 583**
**EP-A2- 1 291 009**
**WO-A1-2009/131736**
**WO-A2-01/74294**
**CN-A- 102 228 419**
**DE-A1-102010 008 321**
**DE-U1- 20 320 413**
**US-A1- 2005 025 727**
**US-A1- 2009 185 989**
**US-A1- 2010 003 201**
**US-B1- 7 172 754**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 771 072 B1

## Description

Field of the invention

The invention relates to a sunscreen composition, especially to one that provides good UVA and UVB sun protection in a silicone elastomer base composition.

Background of the invention

Solar radiation includes about 5 % ultraviolet (UV) radiation, wavelength of which is between 200 nm and 400 nm. It is further classified into three regions: from 320 to 400 nm (UV-A), 290 to 320 nm (UV-B) and from 200 to 290 nm (UV-C). Exposure to UV-A and UV-B radiation for short period is known to cause reddening of the skin and localized irritation. Continued and prolonged exposure can lead to sunburn, melanoma and formation of wrinkles. It is also reported that UV radiation causes significant damage to hair. Therefore people desire to protect their skin and hair from the harmful effects of both UV-A and UV-B radiation.

Various cosmetic preparations have been reported for preventing and/or protecting the skin from harmful effects of ultraviolet radiation. These cosmetic compositions usually comprise different types of organic sunscreen agents, especially the ones capable of absorbing the UV-A and UV-B radiation present in the sun's rays. Thus both UV-A and UV-B sunscreens are usually incorporated in photoprotective compositions so as to provide protection over the entire range of UV radiation. Sun protection Factor (SPF) and UV-A protection factor (UVAPF) are commonly measured attributes of photoprotective compositions which indicate the protection that the skin gets from exposure to both UV-B and UV-A radiation.

Thus consumers now look for products that claim higher and higher SPF/ UVAPF. One of the ways of achieving this is to incorporate high levels of UV-A and UV-B sunscreens, very often higher than 15 % by weight of the sunscreen composition. One disadvantage of this approach is the high cost associated with incorporation of high levels of sunscreens which are expensive. Further, there are safety and regulatory limitations on the upper limit of incorporation of these sunscreens. Sensory properties are also reported to get affected on incorporation of high levels of sunscreens. Yet another problem in incorporating high amount of organic sunscreens is their stability since the sunscreens could interact at these high levels either with each other or their solubility in the composition may be kinetically determined and they may tend to precipitate out on storage. Hence, there is a problem of achieving stable high SPF/ UVAPF compositions while keeping the total amount of sunscreens in the compositions relatively low without loss of desired sensory properties.

The present inventors have been working on achieving high SPF in photoprotective composition comprising a silicone elastomer base, especially in water-in-oil emulsion compositions. The present inventors seek to achieve high SPF/ UVAPF, i.e. higher than 10 SPF and higher than 2 UVAPF values, with use of relatively small amounts of sunscreens. They have been working to invent sunscreen compositions which achieve these high values with total organic sunscreens of less than 15 %, preferably less than 10 %, and optimally less than 7 % by weight of the composition. Heretofore, inclusion of UV-B sunscreens in such compositions were possible with the compositions exhibiting high stability. However when UV-A sunscreens were sought to be incorporated in such compositions, they tended to be unstable in the emulsion and were seen to crystallize out over a period of few weeks or months on storage. The present inventors have unexpectedly determined that UV-A sunscreens could be incorporated along with UV-B sunscreens or a broad spectrum sunscreen having both UV-A and UV-B functionality could be incorporated, at the abovementioned low amounts, to achieve these high SPF/ UVAPF values by including a specific class of emollients meeting a desired solubility parameter along with select class of volatile silicones meeting a desired vapour pressure and heat of vapourisation conditions, in such silicone elastomer based compositions.

Some publications on sunscreen compositions in this field have been reported. US 4 940 574 (Kaplan, 1990) discloses non-aqueous sunscreen oils having a high SPF value and containing a volatile oil such as a volatile silicone oil, an emollient containing esters of straight and branched chain C10-C16 alcohols and C4-C20 mono- or dicarboxylic acids such as tridecyl stearate and an ester of a straight or branched-chain C10-C16 alcohol and a tri(lower alkyl) substituted benzoic acid such as tridecyl trimellitate, esters of branched-chain C5-C10 glycols and C4-C20 mono- and dicarboxylic acid such as the diester of neopentyl glycol and decanoic acid and the diester of neopentyl glycol and octanoic acid and a sunscreening effective amount of at least two UV-B type sunscreens and at least one UV-A type sunscreen. This patent discloses how to achieve high SPF in a non-aqueous system with use of high amounts of total organic sunscreens (above about 17 % by weight of the composition).

US 2009/185989 (Coty, 2009) discloses a cosmetic sun protection product based on water in silicone emulsions which includes 22-32 weight percent of a volatile cyclic silicone oil, 2.0-4.0 weight percent of a non-volatile silicone elastomer, 0.9-2.8 weight percent of an emulsifier, 0.1-0.5 weight percent of an amino acid/fatty acid copolymer, 19-24 weight percent of a mixture of organic UVA and UVB filters at a ratio of from 1:1.5 to 1:1; and cosmetic adjuvants, excipients, active substances and mixtures thereof to make 100 weight percent, the Brookfield viscosity of the sun

protection product ranging from 3,000 to 20,000 mPas. This publication does not teach inclusion of an emollient having specific solubility parameter which in combination with the other ingredients of the present invention provide for the high SPF/ UVAPF.

EP2100583 discloses a cosmetic foundation in the form of a water-in-silicone emulsion characterized in that the preparation comprises (a) at least one particulate photoprotective filter (b) one or more further selected UV photo-protective filter selected from a particular group and (c) one or more dyes and/ or pigments. In this publication, the amount of emollient disclosed is not sufficient to solubilise the oily components thereby providing the high SPF claimed in the present invention.

Therefore there exists a need for a personal care composition comprising sunscreen agents preferably in low concentrations that are able to provide high SPF / UVAPF values in water in silicone oil compositions.

It is therefore an object of the present invention to obviate at least some drawbacks of the prior art and provide high SPF/ UVAPF photo-protective sunscreen composition.

Another object of the present invention is to provide high SPF/ UVAPF sunscreen composition without compromising on the desired skin sensorial properties in water-in-oil emulsion compositions.

## Summary of the invention

The first aspect of the invention provides for an aqueous photo-protective personal care composition comprising less than 15 % w/w total organic sunscreen comprising

(i) 0.1 to 10 % w/w of a UVA sunscreen;
(ii) 0.1 to 10 % w/w of a UVB sunscreen;
(iii) 3 to 50 % w/w of an emollient having a Hansen Solubility Parameter between 12.4 to 22.0 $MPa^{1/2}$ at 25°C selected from the group consisting of (a) ester of alkoxylated aromatic alcohol with fatty carboxylic acid, (b) esters of polyglycols or diols with fatty carboxylic acid such as caprylic/capric triglyceride, (c) ester of fatty alcohol and fatty acid; (d) alkoxylated derivative of benzyl alcohol and mixtures thereof; and
(iv) a cosmetically acceptable base comprising (a) 5 to 95 % of a volatile silicone by weight of said base selected from the group consisting of octa methyl cyclo tetra siloxane, deca methyl cyclo penta siloxane, dodecamethyl cyclo hexa siloxane, blends of methyl trimethicone and dimethicone and mixtures thereof, the volatile silicone having a vapor pressure value at 25°C of 2.6 to 1400 Pa, and a heat of vaporization value of 21 to 84 kJ/mole and (b) 5 to 95% of a silicone elastomer by weight of said base.

According to another aspect of the present invention there is provided an aqueous photo-protective personal care composition comprising

(i) 0.1 to 15 % w/w of an organic sunscreen having a molar absorption coefficient ε at 310 nm (UV-A region) of higher than 10,000 $M^{-1}$ $cm^{-1}$ and molar absorption coefficient at 360 nm (UV-B region) of higher than 10,000$M^{-1}cm^{-1}$;
(ii) 3 to 50 % w/w of an emollient having a Hansen Solubility Parameter between 12.4 to 22.0 $MPa^{1/2}$ at 25°C selected from the group consisting of (a) ester of alkoxylated aromatic alcohol with fatty carboxylic acid, (b) esters of polyglycols or diols with fatty carboxylic acid such as caprylic/capric triglyceride, (c) ester of fatty alcohol and fatty acid; (d) alkoxylated derivative of benzyl alcohol and mixtures thereof; and
(iii)a cosmetically acceptable base comprising (a) 5 to 95 % of a volatile silicone by weight of said base selected from octa methyl cyclo tetra siloxane, deca methyl cyclo penta siloxane, dodecamethyl cyclo hexa siloxane, blends of methyl trimethicone and dimethicone and mixtures thereof, the volatile silicone having a vapor pressure value at 25°C of 2.6 to 1400 Pa, and a heat of vaporization value of 21 to 84 kJ/mole and (b) 5 to 95% of a silicone elastomer by weight of said base.

Another aspect of the invention provides for use of the composition of the first and second aspect for providing an SPF value of at least 10 and a UVAPF value of at least 2.

## Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight

percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "A Photo-protective personal care Composition" as used herein, is meant to include a composition for topical application to sun-exposed areas of the skin and/or hair of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of such sunscreen compositions include leave-on skin lotions, creams, antiperspirants, deodorants, lipsticks, foundations, mascara, sunless tanners and sunscreen lotions and wash-off shampoos, conditioners, shower gels, toilet bars. The composition of the present invention is preferably a leave-on composition. "Skin" as used herein is meant to include skin on the face and body (e.g., neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photoprotection.

By ’A High SPF sunscreen composition’ is meant a composition that has an SPF higher than 10, preferably higher than 12, more preferably higher than 15. By ’A High UVAPF sunscreen composition’ is meant a composition that has a UVAPF equal to or higher than 2, preferably higher than 4.

The SPF and UVAPF are measured using the standard protocols ISO/WD 4445 and ISO/CD 24443 respectively.

The invention provides for an aqueous photo-protective personal care composition comprising less than 15 % total organic sunscreen comprising a UVA sunscreen, a UVB sunscreen, an emollient having a specific Hansen Solubility Parameter from specified classes and a cosmetically acceptable base comprising a volatile silicone having a specific vapor pressure value and a heat of vaporization value selected from specified classes. Instead of two sunscreens, one providing UV-A protection and the other providing UV-B protection, it is also possible to include one sunscreen that provides both functionalities.

The composition of the invention provides excellent results in terms of high SPF / UVAPF values even when the composition comprises less than 10 % total organic sunscreens, and also when it is less the less than 7 % total organic sunscreens.

An example of a sunscreen that has both high UV-A and UV-B sunscreen efficacy is Bis-Ethylhexyloxyphenol Methoxyphenyl Triazine sold under the brand name Tinosorb S from BASF ($\varepsilon_{310nm}$ = 46800 $M^{-1}cm^{-1}$, $\varepsilon_{343nm}$ = 51900 $M^{-1}cm^{-1}$).

The UVA sunscreen is preferably a dibenzoylmethane, triazine, or benzophenone derivative. The more preferred UVA sunscreen is a dibenzoylmethane derivative, the most preferred sunscreen is 4-tert-butyl-4’-methoxydibenzoylmethane. The UVA sunscreen is present in 0.1 to 10 %, preferably 0.1 to 5 %, more preferably 0.4 to 3 % by weight of the composition.

The UV-B sunscreen is preferably selected from the class of benzophenones, anthranilates, salicylates, cinnamates, camphores, benzylidene malonate, triazone or derivatives thereof. Another preferred UV-B sunscreen/stabilizer is from the class of diphenyl acrylic acid derivatives. One commercially available sunscreen from this class is sold as Octocrylene™. The most preferred UV-B sunscreen is from the class of cinnamates preferably 2-ethylhexyl-p-methoxycinnamate. The UVB sunscreen is present in 0.1 to 10 %, preferably 1 to 7 %, more preferably 2 to 5 % by weight of the composition.

It is preferred that the weight ratio of UVA:UVB sunscreen present in the composition of the present invention is in the range of 1:1 to 1:1.5. When the amount of UVA and/ or UVB sunscreen is outside this range it is found that there is a higher tendency for the UVA sunscreen to crystallize out of the composition when present in the higher ranges. It is difficult to obtain the desired balance between SPF and UVAPF values when the UVA sunscreen is present in the lower ranges.

The composition of the invention comprises 3 to 50 % of an emollient having a Hansen Solubility Parameter between 12.4 to 22.0 $MPa^{1/2}$ at 25°C selected from (a) ester of alkoxylated aromatic alcohol with fatty carboxylic acid, (b) esters of polyglycols or diols with fatty carboxylic acid such as caprylic/capric triglyceride, (c) ester of fatty alcohol and fatty acid; (d) alkoxylated derivative of benzyl alcohol and mixtures thereof Examples of these classes and the brand names with which they can be obtained are:

(a) Esters of alkoxylated aromatic alcohol and fatty carboxylic acid

(i) PPG-3 Benzyl Ether Ethyl Hexanoate (commercially available as Crodamol SFX),

(ii) PPG-3 Benzyl Myristate (commercially available as Crodamol STS).

(b) Saturated triglycerides like caprylic/ capric triglycerides (commercially available as Crodamol GTCC).
(c) Ethyl Hexyl Hydroxy Stearate.

Hansen Solubility Parameters are calculated using Hansen Solubility Parameters in Practice (HSPiP) software. This is available for purchase from http://www.hansen-solubility.com/index.php?id=16. The present set of calculations were carried out using version 3.

Specifically, each molecule is given three Hansen parameters, each generally measured in $MPa^{0.5}$:

- $\delta_d$ The energy from dispersion forces between molecules
- $\delta_p$ The energy from dipolar intermolecular force between molecules
- $\delta_h$ The energy from hydrogen bonds between molecules.

These three parameters can be treated as co-ordinates for a point in three dimensions also known as the Hansen space. The nearer two molecules are in this three dimensional space, the more likely they are to dissolve into each other

In the Hansen approach these parts quantitatively describe the nonpolar, atomic (dispersion) interactions, $\mathbf{E_D}$, permanent dipole-permanent dipole molecular interactions, $\mathbf{E_P}$, and the hydrogen bonding (electron interchange) molecular interactions, $\mathbf{E_H}$.

$$Eq.\ 1 \quad E = E_D + E_P + E_H$$

*E* can be experimentally measured by determining the energy required to evaporate the liquid, thus breaking all of its cohesion bonds in the process.

$$Eq.\ 2 \quad E = \Delta H_V - RT$$

***Dividing Equation 1 by the molar volume, V, gives the respective Hansen cohesion energy (solubility) parameters according to Eq. 4***.

$$Eq.\ 3 \quad E/V = (E_D/V) + (E_P/V) + (E_H/V)$$

$$Eq.\ 4 \quad \delta^2 = \delta_D^2 + \delta_P^2 + \delta_H^2$$

The total cohesion energy divided by the molar volume is the total cohesion energy density. The square root of this is the Hildebrand total solubility parameter, $\delta$. The SI units for all of these are $MPa^{½}$.

The emollient is preferably present in higher than 3% but less than 50%, more preferably 4 to 12 %, further more preferably 5 to 7 % by weight of the composition. The Hansen solubility parameter of the emollient is preferably in the range of 16 to19 $MPa^{1/2}$ more preferably 18.2 to 18.8 $MPa^{1/2}$.

The cosmetically acceptable base comprises 5 to 95 % of a volatile silicone by weight of said base, having a vapor pressure value at 25°C of 2.6 to 1400 Pa, and a heat of vaporization value of 21 to 84 kJ/mole. The volatile silicone meeting the above specification of vapour pressure and heat of vaporization ensures that the heat absorbed from the substrate (e.g. skin) is such as to have the desired sensory perception of cooling while ensuring the desired UV protection and composition stability.

The preferred volatile silicones are (a) decamethyl cyclopentasiloxane (commercially known as $D_5$ available from Dow Corning DC245); (b) dodecamethyl cyclohexasiloxane (commercially known as $D_6$ and available from Dow corning); (c) **TMF-1.5** (INCI: Methyl Trimethicone); (d) **DM-Fluids-$A_6$** (INCI: Dimethicone); (e) **DM-Fluid-2cs** (INCI: Dimethicone); (f) **SF1000N1** (INCI: Trisiloxane (1 cSt); (g) **SF1000N1.5** (INCI: Dimethicone (1.5cSt); (h) **SF1000N2** (INCI: Dimethicone (2 cSt); and DM-Fluid- 5cSt (INCI: Dimethicone).

Most prefered volatile silicones are (a) decamethyl cyclopentasiloxane (commercially known as D5 available from Dow Corning DC245; (b) **DM-Fluid-2cs; and (c) DM-Fluid** -5 **cst** (INCI: Dimethicone).

The volatile silicone is preferably present in 10 to 23 %, more preferably in 19 to 22 % by weight of said

composition.

The cosmetically acceptable base comprises 5 to 95%, preferably 5-50 % silicone elastomer, more preferably 10 to 30 % by weight of the base. Silicone elastomer is preferably present in 1 to 15 % by weight of the composition.

Silicone elastomers differ from linear polymers because of cross-linking. Many silicone elastomers are made from linear silicone polymers that contain reactive sites along the polymer chain. Elastomers have very different physical and chemical properties from linear polymers, and the properties of elastomers depend very much on the number of cross-links. An elastomer with a relatively small number of cross-links will be very soft and will swell significantly in the presence of a compatible solvent. As the number of cross-links increases, the hardness of the elastomer increases, and the elastomer will swell to a lesser extent in the presence of solvent. A highly suitable silicone elastomers for use in the composition of the invention is DC 9045, a dimethicone crosspolymer commercially available from Dow Corning. DC 9045 is chemically a blend of cyclopentasiloxane swelling agent and dimethicone crosspolymer (12-13 %).

The swelling agent is most preferably a silicone fluid or a functional silicone fluid. The swelling agent is preferably used in an amount which is in a weight ratio of 1:10 to 10:1, more preferably 1:1 to 5:1 with respect to the reaction mixture where the silicone elastomer is prepared. Swelling agent is most preferably low molecular weight silicone oil which includes (i) low molecular weight linear and cyclic volatile methyl siloxanes, (ii) low molecular weight linear and cyclic volatile and non-volatile alkyl and aryl siloxanes, and (iii) low molecular weight linear and cyclic functional siloxanes. Most preferred, however, are low molecular weight linear and cyclic volatile methyl siloxanes (VMS). By "low molecular weight" in this paragraph is meant a compound having a molecular weight from 1000 to 9000.

Other useful silicone elastomer blends which may be used in the present invention are commercially available as

(i) DC 9027 (a blend of an ultra high viscosity dimethiconol and silicone elastomer in cyclopentasiloxane) available from Dow Corning
(ii) DC 9546 (a blend of high molecular weight silicone elastomer, cyclopentasiloxane and a high molecular weight linear silicone polymer) available from Dow Corning,
(iii) EL8050 (a blend of high molecular weight polyglycol-modified silicone elastomer in isododecane) available from Dow Corning and
(iv) EL8051 (a blend of high molecular weight polyglycol-modified silicone elastomer in isodecyl neopentanoate) available from Dow Corning.
(v) Organomodified silicone elastomer having following chemical structure.

Organomodified silicone elastomer

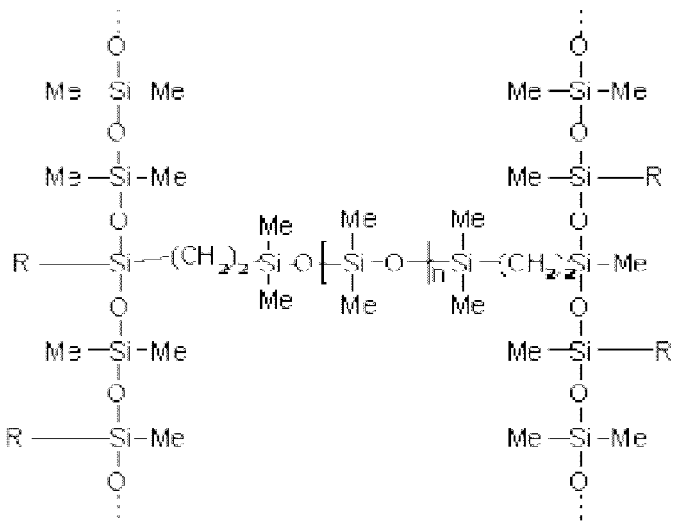

Swollen in mixture of emollient having a Hansen Solubility Parameter between 12.4 to 22.0 MPa1/2 and Volatile silicone and sunscreens

$R = -(CH_2)nCH_3$ and / or R = polyether chains

n = 6 - 30

It is preferred that the composition comprises 0.1 to 5 % oil soluble film forming polymer by weight of the

composition. Suitable film forming polymer is selected from the class of polyacrylates, polyamides, or polyvinyl alcohols which may be defined by the general formula given below:

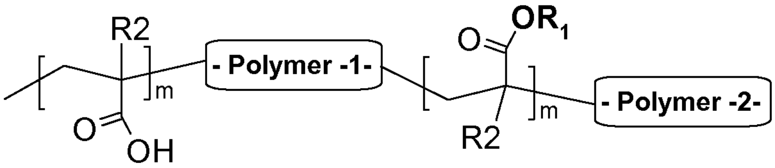

R1 = C1 to C30 chain: For solubilization of sunscreens

Polymer -1 = Film forming polymers: Examples Poly (Acrylates / amides / vinyl alcohols)

Polymer -2 (optional) = Effective spreading aid: Example Silioxane

Tg < 37 deg C R2 = H or Alkyl group

In the above structure R1 may be saturated or unsaturated, branched or cyclic. 'm' may have a value from 5 to 10,000. In the above structure the terminal groups at either end may be a hydroxyl or a carboxyl group.

Examples of film forming polymers or oil thickeners are *Dow Corning*® FA 4002 ID Silicone Acrylate (INCI: Isododecane (and) Acrylates/ Polytrimethylsiloxymethacrylate Copolymer); and c10-30 alkyl acrylate. Most preferred polymers are the one which are capable of thickening oil phase or cosolubilizing oil phase.

The following commercially available polymers were also used in the composition of the invention and found to thicken or cosolubulise the oil phase. Consequently, high SPF was obtained when such polymers / thickeners were used.

(i) Triglyceride of behenic (C22) acid / Tribehenin available as Syncrowax HRC from Croda.

(ii) C30-45 alkyl methicone and C30-45 Olefin available as a cosmetic wax under the brand name AMS C-30 from Dow Corning.

(iii) Hydrogenated soy polyglycerides and C15-23 alkane available as HY 3050 Soy Wax from Dow Corning.

The composition preferably comprises 10 to 70 %, more preferably 30 to 50 % water by weight of the composition. The composition is a water-in-oil composition.

Other useful ingredients usually included in sunscreen compositions may be added for additional benefits. Inorganic sunblocks may be preferably used in the present invention. These include, for example, zinc oxide, iron oxide, silica, such as fumed silica, or titanium dioxide. The total amount of sun block that is preferably incorporated in the composition according to the invention is from 0.1 to 10 % by weight of the composition.

The composition of the invention may additionally comprise a skin lightening agent. The skin lightening agent is preferably chosen from a vitamin B3 compound or its derivative e.g. niacin, nicotinic acid, niacinamide or other well known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10 %, more preferably 0.2 to 5 % by weight of the composition.

The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition of the invention may comprise a conventional deodorant base as the cosmetically acceptable carrier. By a deodorant is meant a product in the stick, or roll-on, which are used for personal deodorant benefit e.g. application in the under-arm or any other area which may or may not contain anti-perspirant actives.

Deodorant compositions can generally be in the form of firm solids, soft solids, gels, creams, and liquids and are dispensed using applicators appropriate to the physical characteristics of the composition.

The compositions of the present invention can comprise a wide range of other optional components. The CTFA Cosmetic Ingredient Handbook, Second Edition, 1992, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

According to another aspect of the present invention there is provided use of a composition of the first aspect of the invention for obtaining SPF of at least 10 and a UVAPF of at least 2. The SPF is preferably higher than 12, more preferably higher than 15. The UVAPF is preferably higher than 4. The use is preferably for non-therapeutic benefits.

The invention is now further described by way of the following non-limiting examples.

**Examples**

Examples 1-4: Advantages of invention as compared to conventional samples

Water-in-oil compositions as shown in 1 were prepared. The samples were stored for 7 days at 25 °C and the samples were observed visually for stability.

Invitro-SPF of the various samples was measured using an Optometrics 290S instrument model. The substrate used was a 8 cm Transpore tape procured from 3M Company. The sample was applied at 2 mg/cm$^2$.

The in-vitro method employed for determination of the UVA-PF has been developed by COLIPA. In order to simulate this method, the film irregularity model was not used, but a relationship between parent film extinction and extinction of the film on the substrate was used. Thus, an in-vitro extinction spectrum is simulated, resulting in a simulated in-vitro UVA-PF. The visual appearance of the samples were observed and the SPF and UVAPF of the samples were measured and are summarized in table 1

Table 1

| **Ingredient** | **Ex 1** | **Ex 2** | **Ex 3** | **Ex 4** |
|---|---|---|---|---|
| Dimethicone Crosspolymer as an ingredient of DC9045# | 3.4 | 3.4 | 3.4 | 3.4 |
| Deca methyl cyclo penta siloxane (D5) as an ingredient of DC9045# | 22 | 22 | 22 | 22 |
| Caprylyl Methicone | 0.50 | 0.50 | 0.50 | 0.50 |
| PEG-10 Dimethicone | 1.18 | 1.18 | 1.18 | 1.18 |
| Mica & titanium dioxide | 0.50 | 0.50 | 0.50 | 0.50 |
| Titanium dioxide(CI 77891) dimethicone, aluminum hydroxide | 0.60 | 0.60 | 0.60 | 0.60 |
| Glycerine | 7.25 | 7.25 | 7.25 | 7.25 |
| Titanium dioxide (micro fine) | 2.00 | 2.00 | 2.00 | 2.00 |
| Emollient as per invention PPG-3 Benzyl Ether Ethylhexanoate (Crodamol SFX) Hansen solubility parameter =18.2 MPa$^{1/2}$ | - | - | 6.00 | - |
| Emollient as per invention Caprylic / Capric Triglycerides Hansen solubility parameter =18.8 MP1/2 | - | - | - | 6.00 |
| Solubilizers: X-tend (phenethyl benzoate) | - | 6.00 | - | - |
| UVA sunscreen 4-t-butyl, 4'-methoxydibenzoylmethane | 1.50 | 1.50 | 1.50 | 1.50 |
| UVB Sunscreen 2-ethylhexyl-p-methoxycinnamate | 4.50 | 4.50 | 4.50 | 4.50 |
| Deionized Water | To 100 | To 100 | To 100 | To 100 |
| Observation | Crystals observed | Immiscible phases | No crystals or | No crystals or |

(continued)

| Ingredient | Ex 1 | Ex 2 | Ex 3 | Ex 4 |
|---|---|---|---|---|
| | on 7 days storage | observed on storage | immiscible phases observed | immiscible phases observed |
| In-vitro SPF | 11 | 18 | 28 | 33 |
| UVAPF | 2.8 | 6.5 | 7.7 | 8.8 |
| #DC 9045 (INCI Name: Cyclopentasiloxane (and) Dimethicone Crosspolymer) is a mixture of high molecular weight silicone elastomers (dimethicone crosspolymer) in cyclopentasiloxane. | | | | |

The data in table 1 indicates that the compositions as per the invention (examples 3 and 4) are not only very stable but also provides high SPF and UVAPF values as compared to a conventional composition (example 1) or a composition where a conventional emollient/solubiliser is used (example 2)

Example 5 and 6: Advantage of using the composition of the invention compared to an anhydrous composition

Compositions as shown in table 2 below were prepared. The SPF of the compositions were measured using the procedure similar to that of example 1 to 4. The data is shown in table 2.

Table 2

| Example 5<br>Composition as per the invention | Example 6<br>Composition similar to that disclosed in US4940574 |
|---|---|
| DC9045 (26.5 %) containing silicone elastomer at 3.5 % and D5 at 23 % | Neopentyl glycol Dicaprylate/Dicaprate & Tridecyl Stearate & Tridecyl Trimellitate (30 %) |
| UVA-Parsol 1789 (2 %) | Silicone Fluid DC344 (38.7 %) |
| UVB-MCX (5 %) | Octadecene (3 %) |
| Capric/Caprylic Triglycerides (5 %) | Fragrance (1.5 %) |
| DC 245 Fluid (0.5 %) | UVB -MCX (7.5 %) |
| Glycerin (7.25 %) | UVB &UVA-Benzophenone-3 (6 %) |
| Niacinamide (3 %) | UV-B PABA (8 %) |
| Ammonium Lactate(5.7 %) | UVB-Homo menthyl Salicylate (5 %) |
| Water (to 100 %) | Propyl Paraben (0.1 %) |
| | Benzoic Acid (0.2 %) |
| SPF = 32 | SPF = 15 |

The data in table 2 indicates that the composition as per the invention (example 5) provides for vastly superior SPF as compared to an anhydrous composition (example 6) that comprises emollients and volatile silicones and with high amount of sunscreens.

Examples 7-10: Use of polymers in the composition of the invention to thicken/ co-solublise thus providing high SPF.

Compositions similar to Example 4 were prepared except that additionally 2 % of a polymer was included. The resultant SPF is summarized in Table 3.

Table 3

| Example | Polymer used | SPF |
|---|---|---|
| 4 | No additional polymer | 31.1 |

(continued)

| Example | Polymer used | SPF |
|---|---|---|
| 7 | C10-C30 alkyl acrylate | 44.4 |
| 8 | Syncrowax HRC | 43.3 |
| 9 | AMS C-30 Cosmetic wax | 47.9 |
| 10 | HY 3050 | 45.1 |

The data in Table 3 indicates that inclusion of select polymers provides for high SPF in the composition of the invention.

Examples 11-15: Stability of suncreens in the composition of the invention

Compositions similar to Example 4 were prepared except that various other organic UV-A and UV-B sunscreens were used or a single broad spectrum UV sunscreen was used. The % UV-A and UV-B sunscreen remaining after UV exposure was measured and the data is summarized in Table 4. The general procedure used to measure the sunscreen concentration is given below:

Skin cream was applied on a PMMA plate. % Transmittance was measured before the solar exposure (0 min). Same plate was exposed to solar simulated light (% Transmittance was measured after 30 minutes of sun exposure. Average absorbance at 310 & 355 nm was recorded to measure % UVA and UVB photostability respectively. UV radiation for exposure was generated using a SPF-290S SPF meter (Optometrics Corporation).

Table 4

| Example | Sunscreens used (wt%) | % UVA remaining | %UVB remaining |
|---|---|---|---|
| 4 | Parsol MCX (5%) + Parsol 1789 (2%) | 37.5 | 49.9 |
| 11 | Uvinul A (2%) + Parsol MCX (5%) | 97.3 | 85.8 |
| 12 | EHMC (4%) + Parsol MCX (5%) | 98.7 | 95.9 |
| 13 | Parsol MCX (5%) + Parsol 1789 (2%) + Tinosorb S (5%) | 91.4 | 91.9 |
| 14 | Parsol 1789 (2%) + Octocrylene (10%) | 97.9 | 99.1 |
| 15 | Tinosorb S (5%) | 80.3 | 78.8 |

The chemical names of the organic sunscreens used in the above table are as follows:

$\varepsilon$ refers to the molar absorption coefficient and has the units of $M^{-1}$ $cm^{-1}$.
Parsol 1789: 4-t-butyl, 4'-methoxydibenzoylmethane; $\varepsilon_{360}$nm = 31, 000 $M^{-1}cm^{-1}$ sourced from Chem Spec);
Parsol MCX: Octyl methoxycinnamate; $\varepsilon_{310\ nm}$ = 24,200 $M^{-1}cm^{-1}$, sourced from Chem Spec);
Octocrylene: 2-ethylhexyl 2-cyano-3,3- diphenyl-2-propenoate ($\varepsilon_{310\ nm}$ = 12,600 $M^{-1}cm^{-1}$, sourced from Merck);
Tinosorb S: Bis-Ethylhexyloxyphenol Methoxyphenyl Triazine (, $\varepsilon_{310nm}$ = 46800 $M^{-1}cm^{-1}$, $\varepsilon_{343\ nm}$ = 51900 $M^{-1}cm^{-1}$, sourced from BASF);
Uvinul A: Diethylamino Hydroxybenzoyl Hexyl Benzoate $\varepsilon_{354nm}$ = 35900 $M^{-1}cm^{-1}$ sourced from BASF);
EHMC: Ethylhexyl **methoxycrylene ;** $\varepsilon_{max}$, 345 nm = 12,600 $M^{-1}cm^{-1}$ sourced from Hallstar).

The data in Table 4 above indicates that highly stable sunscreen compositions can be prepared as per the invention using various combinations of UV-A and UV-B sunscreens or using a single broad spectrum UV sunscreen.
The invention thus provides for a high SPF/UVAPF sunscreen composition comprising relatively low amount of sunscreen compounds in a water in oil emulsion composition that is highly stable.

## Claims

1. An aqueous photo-protective personal care composition having SPF higher than 10 and a UVAPF equal to or higher than 2, comprising less than 15 % w/w total organic sunscreen comprising

   (i) 0.1 to 10 % w/w of a UVA sunscreen which is a dibenzoylmethane;
   (ii) 0.1 to 10 % w/w of a UVB sunscreen;
   (iii) 3 to 50 % w/w of an emollient having a Hansen Solubility Parameter between 12.4 to 22.0 $MPa^{1/2}$ at 25°C selected from the group consisting of (a) ester of alkoxylated aromatic alcohol with fatty carboxylic acid, (b) esters of polyglycols or diols with fatty carboxylic acid such as caprylic/capric triglyceride, (c) ester of fatty alcohol and fatty acid; (d) alkoxylated derivative of benzyl alcohol and mixtures thereof ;
   (iv) 5 to 70% of a cosmetically acceptable base comprising (a) 5 to 95 % of a volatile silicone by weight of said base selected from octa methyl cyclo tetra siloxane, deca methyl cyclo penta siloxane, dodecamethyl cyclo hexa siloxane, blends of methyl trimethicone and dimethicone and mixtures thereof, the volatile silicone having a vapor pressure value at 25°C of 2.6 to 1400 Pa, and a heat of vaporization value of 21 to 84 kJ/mole and (b) 5 to 95% of a silicone elastomer by weight of said base;
   (v) 10 to 70% w/w/ water.

2. A composition as claimed in claim 1 comprising less than 10 % w/w total organic sunscreens.

3. A composition as claimed in claim 2 comprising less than 7 % w/w total organic sunscreens.

4. A composition as claimed in claim 1 wherein said dibenzoylmethane is 4-tert-butyl-4'-methoxydibenzoylmethane.

5. A composition as claimed in any one of the preceding claims wherein the UV-B sunscreen is selected from the group consisting of a benzophenone, a anthranilate, a salicylate, a cinnamate, a camphore, benzylidene malonate, a triazone, and derivatives thereof.

6. A composition as claimed in any one of the preceding claims wherein said cosmetically acceptable base comprises 5-50 % silicone elastomer by weight of said base.

7. A composition as claimed in any one of the preceding claims comprising 0.1 to 5 % w/w oil soluble film forming polymers selected from the group consisting of a polyacrylate, a polyamide, and a polyvinyl alcohol.

8. A composition as claimed in any one of the preceding claims for use in providing an SPF value of at least 10 and a UVAPF value of at least 2.

## Patentansprüche

1. Wässrige Körperpflegezusammensetzung mit Lichtschutz mit einem SPF von mehr als 10 und einem UVAPF von gleich oder mehr als 2, umfassend weniger als 15% Gew./Gew. gesamtes organisches Sonnenschutzmittel, umfassend

   (i) 0,1 bis 10% Gew./Gew. eines UVA-Sonnenschutzmittels, welches ein Dibenzoylmethan ist,
   (ii) 0,1 bis 10% Gew./Gew. eines UVB-Sonnenschutzmittels,
   (iii) 3 bis 50% Gew./Gew. eines Erweichungsmittels mit einem Hansen-Löslichkeitsparameter zwischen 12,4 und 22,0 $MPa^{1/2}$ bei 25°C, ausgewählt aus der aus (a) Ester von alkoxyliertem aromatischem Alkohol mit Fettcarbonsäuren, (b) Estern von Polyglykolen oder Diolen mit Fettcarbonsäure, wie Capryl/Caprin-Triglycerid, (c) Ester von Fettalkohol und Fettsäure, (d) alkoxyliertem Derivat von Benzylalkohol und Mischungen davon bestehenden Gruppe ausgewählt ist,
   (iv) 5 bis 70% eines kosmetisch verträglichen Grundmaterials, umfassend (a) 5 bis 95% flüchtiges Silikon, bezogen auf das Gewicht des Grundmaterials, ausgewählt aus Octamethylcyclotetrasiloxan, Decamethylcyclopentasiloxan, Dodecamethylcyclohexasiloxan, Mischungen von Methyltrimethicone und Dimethiocone und Mischungen davon, wobei das flüchtige Silicon einen Dampfdruckwert bei 25°C von 2,6 bis 1400 Pa und einen Verdampfungswärmewert von 21 bis 84 kJ/mol und (b) 5 bis 95% Silikon-Elastomer, bezogen auf das Gewicht des Grundmaterials, aufweist,
   (v) 10 bis 70% Gew./Gew. Wasser.

2. Zusammensetzung, wie im Anspruch 1 beansprucht, umfassend weniger als 10% Gew./Gew. gesamte organische Sonnenschutzmittel.

3. Zusammensetzung, wie im Anspruch 2 beansprucht, umfassend weniger als 7% Gew./Gew. gesamte organische Sonnenschutzmittel.

4. Zusammensetzung, wie im Anspruch 1 beansprucht, wobei das Dibenzoylmethan 4-tert.-Butyl-4'-methoxydibenzoylmethan ist.

5. Zusammensetzung, wie in irgendeinem der vorhergehenden Ansprüche beansprucht, wobei das UVB-Sonnenschutzmittel aus der aus einem Benzophenon, einem Anthranilat, einem Salicylat, einem Cinnamat, einem Kampher, Benzylidenmalonat, einem Triazon und Derivaten davon bestehenden Gruppe ausgewählt ist.

6. Zusammensetzung, wie in irgendeinem der vorhergehenden Ansprüche beansprucht, wobei das kosmetisch verträgliche Grundmaterial 5-50% Silikon-Elastomer, bezogen auf das Gewicht des Grundmaterials, umfasst.

7. Zusammensetzung, wie in irgendeinem der vorhergehenden Ansprüche beansprucht, umfassend 0,1 bis 5% Gew./Gew. öllösliche filmbildende Polymere, ausgewählt aus der aus einem Polyacrylat, einem Polyamid und einem Polyvinylalkohol bestehenden Gruppe.

8. Zusammensetzung, wie in irgendeinem der vorhergehenden Ansprüche beansprucht, zur Verwendung in der Bereitstellung eines SPF-Wertes von mindestens 10 und eines UVAPF-Wertes von mindestens 2.

## Revendications

1. Composition aqueuse photo-protectrice pour le soin de la personne ayant un SPF supérieur à 10 et un UVAPF supérieur ou égal à 2, comprenant moins de 15 % en masse/masse d'écran solaire organique total comprenant

   (i) 0,1 à 10 % en masse/masse d'un écran solaire UVA qui est un dibenzoylméthane ;
   (ii) 0,1 à 10 % en masse/masse d'un écran solaire UVB ;
   (iii) 3 à 50 % en masse/masse d'un émollient ayant un paramètre de solubilité Hansen de 12,4 à 22,0 $MPa^{1/2}$ à 25°C choisi dans le groupe constitué de (a) un ester d'alcool aromatique alcoxylé avec un acide carboxylique gras, (b) des esters de polyglycols ou de diols avec un acide carboxylique gras, tel qu'un triglycéride caprylique/caprique, (c) un ester d'alcool gras et d'acide gras ; (d) un dérivé alcoxylé d'alcool benzylique et des mélanges de ceux-ci ;
   (iv) 5 à 70 % d'une base cosmétiquement acceptable comprenant (a) 5 à 95 % d'un silicone volatile en masse de ladite base choisi parmi l'octaméthylcyclotétrasiloxane, le décaméthylcyclopentasiloxane, le dodécaméthylcyclohexasiloxane, des combinaisons de méthyle triméthicone et diméthicone et des mélanges de ceux-ci, le silicone volatile ayant une valeur de pression de vapeur à 25°C de 2,6 à 1 400 Pa, et une valeur de chaleur de vaporisation de 21 à 84 kJ/mole et (b) 5 à 95 % d'un élastomère de silicone en masse de ladite base ;
   (v) 10 à 70 % en masse/masse d'eau.

2. Composition selon la revendication 1 comprenant moins de 10 % en masse/masse d'écrans solaires organiques totaux.

3. Composition selon la revendication 2 comprenant moins de 7 % en masse/masse d'écrans solaires organiques totaux.

4. Composition selon la revendication 1, dans laquelle ledit dibenzoylméthane est le 4-tert-butyl-4'-méthoxydibenzoylméthane.

5. Composition selon l'une quelconque des revendications précédentes, dans laquelle l'écran solaire UV-B est choisi dans le groupe constitué d'une benzophénone, d'un anthranilate, d'un salicylate, d'un cinnamate, d'un camphre, de malonate de benzylidène, d'une triazone, et de dérivés de ceux-ci.

6. Composition selon l'une quelconque des revendications précédentes, dans laquelle ladite base cosmétiquement acceptable comprend 5-50 % d'élastomère de silicone en masse de ladite base.

7. Composition selon l'une quelconque des revendications précédentes comprenant 0,1 à 5 % en masse/masse de polymères formant un film soluble dans l'huile choisis dans le groupe constitué d'un polyacrylate, d'un polyamide, et d'un poly(alcool vinylique).

8. Composition selon l'une quelconque des revendications précédentes pour une utilisation dans la fourniture d'une valeur SPF d'au moins 10 et d'une valeur UVAPF d'au moins 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4940574 A, Kaplan
- US 2009185989 A, Coty
- EP 2100583 A


### Non-patent literature cited in the description


- The CTFA Cosmetic Ingredient Handbook. 1992